# EUROPEAN PATENT SPECIFICATION

(11) **EP 3 368 150 B1**
(45) Date of publication and mention of the grant of the patent: **07.07.2021**
(21) Application number: 16790855.7
(22) Date of filing: 26.10.2016
(51) Int. Cl.: A61N 1/368, A61N 1/37

(54) **MULTISITE PACING CAPTURE DETERMINATION BASED ON EVOKED RESPONSE**
BESTIMMUNG DER ERFASSUNG VON MEHRSTELLIGER STIMULATION AUF BASIS VON AUSGELÖSTER REAKTION
DÉTERMINATION DE CAPTURE DE STIMULATION MULTISITE BASÉE SUR UNE RÉPONSE ÉVOQUÉE

(30) Priority: 30.10.2015 US 201562248683 P
(43) Date of publication of application: 05.09.2018
(73) Proprietor: Cardiac Pacemakers, Inc., St. Paul, MN 55112-5798 (US)
(72) Inventor: SAHA, Sunipa, Shoreview, Minnesota 55126 (US); HERRMANN, Keith L., Minneapolis, Minnesota 55406 (US); YU, Yinghong, Shoreview, Minnesota 55126 (US)
(74) Representative: Pfenning, Meinig & Partner mbB
(86) International application number: PCT/US2016/058856
(87) International publication number: WO 2017/075048

(56) References cited:
- US-A1- 2013 030 492
- US-B1- 6 640 136
- US-B1- 7 899 536
- US-B1- 8 666 490

## Description

### BACKGROUND

Ambulatory medical devices can be used to treat patients or subjects using electrical or other therapy, or to aid a physician or caregiver in patient diagnosis through internal monitoring of a patient's condition. Some types of ambulatory medical devices may be implantable or partially implantable. Some examples include cardiac function management (CFM) devices such as implantable pacemakers, implantable cardioverter defibrillators (ICDs), cardiac resynchronization therapy devices (CRTs), and devices that include a combination of such capabilities. The devices may include one or more electrodes in communication with one or more sense amplifiers to monitor electrical heart activity within a patient, and often include one or more sensors to monitor one or more other internal patient parameters. The devices may be implanted subcutaneously and may include electrodes that are able to sense cardiac signals without being in direct contact with the patient's heart. Other examples of implantable medical devices (IMDs) include implantable diagnostic devices, implantable drug delivery systems, or implantable devices with neural stimulation capability (e.g., vagus nerve stimulator, baroreflex stimulator, carotid sinus stimulator, deep brain stimulator, sacral nerve stimulator, etc.).

Operation of an IMD is typically optimized for particular patient by a caregiver, such as by programming different device operating parameters or settings for example. Manufacturers of such devices continue to improve and add functionality to the devices, which can make them complicated to program. The inventors have recognized a need for improved optimization of device-based therapy.

For example, document US 6,640,136 B1 describes an implantable cardiac stimulation device with automatic electrode selection for avoiding cross-chamber stimulation. The implantable device is intended to stimulate one or more cardiac chambers, for example, in a dual-chamber or multi-chamber device. According to one illustrative embodiment of the device, capture thresholds are determined for a first chamber that is intended to be captured, and also for a second chamber that is not intended to be captured. The stimulation energy is then set to a level based upon the respective capture thresholds.

Document US 8,666,490 B1 describes a medical device which includes pacing circuitry configured to deliver during a cardiac cycle a first pacing pulse to a first site using a first electrode as a cathode electrode for the first pacing pulse and to deliver a second pacing pulse to a second site using a second electrode as a cathode electrode for the second pacing pulse. Sensing circuitry is configured to generate a differential signal between cardiac electrical activity sensed at the first electrode and cardiac electrical activity sensed at the second electrode. Capture circuitry discriminates between single site capture and multi-site capture based on the differential signal.

### OVERVIEW

The invention is defined in independent claims 1 and 11. Preferred embodiments are defined by the dependent claims.
Methods described hereinafter do not form part of the present invention.

The present subject matter relates to providing multi-site electrical stimulation therapy. An example multi-site electrical stimulation therapy is multi-site pacing stimulation delivered to multiple sites within the same chamber of the heart.

An apparatus in accordance with the present invention is for electrical coupling to a plurality of implantable electrodes. The apparatus example includes a stimulus circuit, a cardiac signal sensing circuit, and a control circuit. The stimulus circuit provides electrical pulse energy to at least a first pacing channel that includes a first left ventricular (LV) electrode as a cathode and a second pacing channel that includes a second LV electrode as a cathode. The cardiac signal sensing circuit senses cardiac activity signals using at least a first sensing channel that includes one of the first LV electrode or the second LV electrode. The control circuit includes a capture detection sub-circuit configured to: initiate delivery of electrical pulse energy to both the first pacing channel and the second pacing channel; sense cardiac depolarization of a ventricle using the first sensing channel; determine a first cardiac capture pulse energy level threshold for the first pacing channel; determine a second cardiac capture pulse energy level threshold for the second pacing channel; and provide indications of the first and second cardiac capture pulse energy level thresholds to a user or process. The capture detection sub-circuit is configured to initiate a change in pulse energy level of the electrical pulse energy delivered to both the first pacing channel and the second pacing channel. The control circuit includes a signal processing sub-circuit configured to identify a change in morphology in a cardiac activity signal sensed in association with a change from a first level of electrical pulse energy delivered to both the first pacing channel and the second pacing channel to a second level of electrical pulse energy delivered to both the first pacing channel and the second pacing channel. The capture detection sub-circuit is further configured to distinguish, using the identified change in morphology, between cardiac capture by one of the first and second pacing channels from cardiac capture by both pacing channels.

In accordance with the invention, a machine-readable medium includes instructions that, when performed by the aforementioned apparatus, cause the apparatus to perform acts comprising: delivering electrical pulse energy to both of at least a first pacing channel of the apparatus that includes a first left ventricular (LV) electrode as a cathode and a second pacing channel of the apparatus that includes a second LV electrode as a cathode; sensing cardiac depolarization of a ventricle using at least a first sensing channel that includes one of the first LV electrode or the second LV electrode; changing a pulse energy level of the electrical pulse energy delivered to both of the first and second pacing channel; identifying a change in morphology in a cardiac activity signal sensed in association with a change from a first level of electrical pulse energy delivered to both the first pacing channel and the second pacing channel to a second level of electrical pulse energy delivered to both the first pacing channel and the second pacing channel; distinguishing, using the identified change in morphology, cardiac capture by one of the first and second pacing channels from cardiac capture by both pacing channels; determining a first cardiac capture pulse energy level threshold for the first pacing channel, and a second cardiac capture pulse energy level threshold for the second pacing channel; and providing indications of the first and second cardiac capture pulse energy level thresholds to a user or process.

Multi-site pacing of a heart chamber may add complexity to the programming of parameter values for electrical stimulation therapy. Device generated recommendations for values of parameters associated with the electrical stimulation may assist the clinician or physician to optimize the device for the needs of a specific patient.

This section is intended to provide a brief overview of subject matter of the present patent application. It is not intended to provide an exclusive or exhaustive explanation of the invention. The scope of the present invention is defined by the appended claims.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, the various examples discussed in the present document.
FIG. 1 is an illustration of an example of portions of a system that includes an implantable medical device.
FIGS. 2A and 2B illustrate examples of sensing channels for an ambulatory medical device.
FIG. 3 shows an example of a method of controlling operation of an implantable medical device
FIG. 4 is a block diagram of portions of an example of a medical device to determine cardiac capture pulse energy level thresholds for multi-site pacing.
FIG. 5 illustrates portions of an example of a medical device system that includes electrodes and an implantable medical device configured for dual-site pacing.
FIG. 6 is a flow diagram of an example of a device-based method of determining pacing capture thresholds for multi-site pacing.
FIG. 7 illustrates portions of an example of a medical device system that includes a shared cathode sensing channel.
FIG. 8 is a graph used to illustrate the cardiac capture threshold determination of the method example of FIG. 6.
FIGS. 9A and 9B show an example of a shift in sensed cardiac signal morphology from shared cathode sensing to independent pacing and sensing.
FIG. 10 is a graph used to illustrate the cardiac capture threshold determination of the method example of FIG. 6.
FIG. 11 is a flow diagram of another example of a device-based method of determining pacing capture thresholds for multi-site pacing.
FIG. 12 is an illustration of portions of an example of a system that uses a deployed implantable medical device to provide a therapy to a patient.

### DETAILED DESCRIPTION

An ambulatory medical device can include one or more of the features, structures, methods, or combinations thereof described herein. For example, a cardiac function management device may be implemented to include one or more of the advantageous features or processes described below. It is intended that such a cardiac function management device, or other implantable or partially implantable device, need not include all of the features described herein, but may be implemented to include selected features that provide for unique structures or functionality. Such a device may be implemented to provide a variety of therapeutic or diagnostic functions.

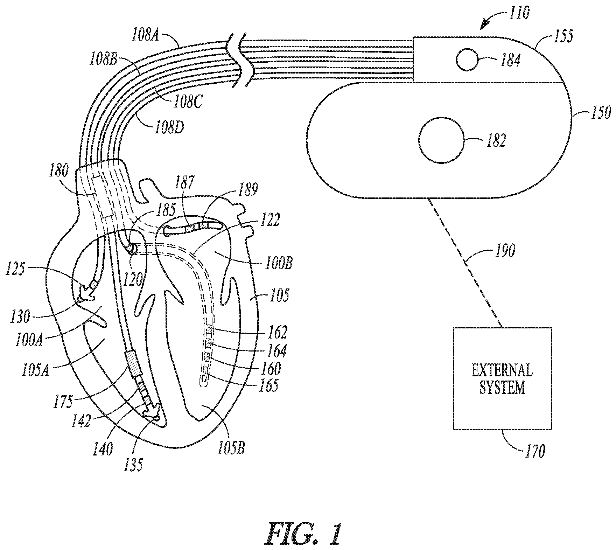

FIG. 1 is an illustration of portions of a system that includes an ambulatory medical device that is an IMD 110. Examples of IMD 110 include, without limitation, a pacemaker, a defibrillator, a cardiac resynchronization therapy (CRT) device, or a combination of such devices. In other examples, the IMD is a neurostimulator such as among other things a vagus nerve stimulator, baroreflex stimulator, carotid sinus stimulator, deep brain stimulator, or sacral nerve stimulator. The system 100 also typically includes an IMD programmer or other external system 170 that communicates wireless signals 190 with the IMD 110, such as by using radio frequency (RF) or other telemetry signals.

The IMD 110 can be coupled by one or more leads 108A-D to heart 105. Cardiac leads 108A-D include a proximal end that is coupled to IMD 110 and a distal end, coupled by electrical contacts or "electrodes" to one or more portions of a heart 105. The electrodes typically deliver cardioversion, defibrillation, pacing, or resynchronization therapy, or combinations thereof to at least one chamber of the heart 105. The electrodes may be electrically coupled to sense amplifiers to sense electrical cardiac signals. Sometimes the sensing circuits and electrodes are referred to as sensing channels. For example, circuitry used to sense signals in an atrium is referred to as an atrial sensing channel, and circuitry used to sense signals in a ventricle is referred to as a ventricular sensing channel. When direction is taken into account due to position of one or more sensing electrodes, the sensing channel can be referred to as a sensing vector.

Sensed electrical cardiac signals can be sampled to create an electrogram. An electrogram can be analyzed by the IMD and/or can be stored in the IMD and later communicated to an external device where the sampled cardiac signals can be displayed for analysis.

Heart 105 includes a right atrium 100A, a left atrium 100B, a right ventricle 105A, a left ventricle 105B, and a coronary sinus 120 extending from right atrium 100A. Right atrial (RA) lead 108A includes electrodes (electrical contacts, such as ring electrode 125 and tip electrode 130) disposed in an atrium 100A of heart 105 for sensing signals, or delivering pacing therapy, or both, to the atrium 100A. Electrodes used to provide pacing therapy can be referred to as pacing channels. When direction is taken into account due to position of one or more pacing electrodes, the pacing channel can be referred to as a pacing vector. Similarly, electrodes used to sense cardiac signals with sense amplifiers can be referred to as sensing channels, and when direction is taken into account a sensing channel can be referred to as a sensing vector.

Right ventricular (RV) lead 108B includes one or more electrodes, such as tip electrode 135 and ring electrode 140, for sensing signals, delivering pacing therapy, or both sensing signals and delivering pacing therapy. RV lead 108B can include one or more additional ring electrodes 142 to provide multi-site pacing to the RV. Lead 108B optionally also includes additional electrodes, such as electrodes 175 and 180, for delivering atrial cardioversion, atrial defibrillation, ventricular cardioversion, ventricular defibrillation, or combinations thereof to heart 105. Such electrodes typically have larger surface areas than pacing electrodes in order to handle the larger energies involved in defibrillation. Lead 108B optionally provides resynchronization therapy to the heart 105. Resynchronization therapy is typically delivered to the ventricles in order to better synchronize the timing of depolarizations between ventricles.

The IMD 110 can include a third cardiac lead 108C attached to the IMD 110 through the header 155. The third cardiac lead 108C includes electrodes 160, 162, 164, and 165 placed in a coronary vein 122 lying epicardially on the left ventricle (LV) 105B via the coronary vein. The number of electrodes shown in the Figure is only an example and other arrangements are possible. For instance, the third cardiac lead 108C may include less electrodes (e.g., one or two electrodes) or more electrodes (e.g., eight or more electrodes) than the example shown, and may include a ring electrode 185 positioned near the coronary sinus (CS) 120. LV lead 108C can provide multi-site pacing to the LV.

In addition to cardiac leads 108A, 108B, 108C, or in the alternative to one or more of cardiac leads 108A, 108B, 108C, the IMD 110 can include a fourth cardiac lead 108D that includes electrodes 187 and 189 placed in a vessel lying epicardially on the left atrium (LA) 100B.

The IMD 110 can include a hermetically-sealed IMD housing or can 150, and the IMD 110 can include an electrode 182 formed on the IMD can 150. The IMD 100 may include an IMD header 155 for coupling to the cardiac leads, and the IMD header 155 may also include an electrode 184. Cardiac pacing therapy can be delivered in a unipolar mode using the electrode 182 or electrode 184 and one or more electrodes formed on a lead. Cardiac pacing therapy can be delivered in an extended bipolar pacing mode using only one electrode of a lead (e.g., only one electrode of LV lead 108C) and one electrode of a different lead (e.g., only one electrode of RV lead 108B). Cardiac pacing therapy can be delivered in a monopolar pacing mode using only one electrode of a lead without a second electrode.

Lead 108B can include a defibrillation RV coil electrode 175 located proximal to tip and ring electrodes 135, 140, and a second defibrillation coil electrode 180 located proximal to the RV coil electrode 175, tip electrode 135, and ring electrode 140 for placement in the superior vena cava (SVC). In some examples, high-energy shock therapy is delivered from the first or RV coil 175 to the second or SVC coil 180. In some examples, the SVC coil 180 is electrically tied to the electrode 182 formed on the IMD can 150. This improves defibrillation by delivering current from the RV coil electrode 175 more uniformly over the ventricular myocardium. In some examples, the therapy is delivered from the RV coil 175 only to the electrode 182 formed on the IMD can 150. In some examples, the coil electrodes 175, 180 are used in combination with other electrodes for sensing signals.

Note that the specific arrangement of leads and electrodes shown in the illustrated example of FIG. 1 is intended to be non-limiting. An IMD can be configured with a variety of electrode arrangements including transvenous, endocardial, and epicardial electrodes (e.g., an epicardial patch that may include dozens of electrodes), and/or subcutaneous, non-intrathoracic electrodes. An IMD 110 can be connectable to subcutaneous array or lead electrodes (e.g., non-intrathoracic electrodes or additional LV leads implantable along the LV wall, and leads implantable in one or both atria) that can be implanted in other areas of the body to help "steer" electrical currents produced by IMD 110. An IMD can be leadless (e.g., a leadless pacemaker). A leadless IMD may be placed in a heart chamber (e.g., RV or LV) and multiple electrodes of the leadless IMD may contact multiple tissue sites of the heart chamber.

Electrical pacing therapy is provided by a CRM device in response to an abnormally slow heart rate to induce cardiac depolarization and contraction (sometimes called capture of the heart, or cardiac capture). Pacing stimulation energy is delivered to provide a depolarization rate that improves hemodynamic function of the patient. The pacing stimulation energy should be optimized to produce cardiac capture. If the pacing stimulation energy is too high, stimulation to multiple pacing sites may cause stress on the heart and the battery life of an implanted device will be needlessly short. Also, pacing stimulation energy that is too high may cause unwanted non-cardiac stimulation such as stimulation of the phrenic nerve or stimulation of muscle near the tissue pocket in which the device is implanted. If the pacing stimulation energy is too low, the pacing energy will not evoke a response in the heart and the device will not produce the desired heart contractions.

To determine the appropriate electrostimulation energy, a CRM device may deliver a sequence of electrostimulation pulses to the heart as part of an automatic cardiac capture test. The sequence may include a successive reduction of the energy of the electrostimulation pulses. A first electrostimulation pulse that will likely induce cardiac capture is delivered. The level of energy of subsequent electrostimulation pulses is reduced in steps until the device verifies that failure to induce capture has occurred. Alternatively, the sequence may include increasing the energy level of the electrostimulation pulses. A first electrostimulation pulse that is below a threshold likely to induce capture is delivered. The energy of subsequent electrostimulation pulses is increased in steps until the device verifies that capture was induced. In another approach, the CRM device may increase and decrease the sequence of stimulation pulses as part of the cardiac capture test.

As explained previously herein, the CRM device may sense cardiac signals using one or more of the sensing channels. To verify cardiac capture, the device may be able to distinguish an electrical signal that shows an evoked response from an electrical that shows no evoked response. Correctly identifying evoked response in the cardiac tissue target can be used detect a change from no capture to capture, or a change from capture to loss of capture.

When the minimum energy required to cause capture of the tissue target is determined, a safety margin can be added to the minimum energy to ensure efficacy of the stimulation pulses. An approach to determining a safety margin for device delivery of electrical pacing therapy can be found in Brisben et al., U.S. Patent No. 8,565,879, "Method and Apparatus for Pacing Safety Margin," filed March 25, 2011.

As explained previously herein, a CRM device may be configured to provide multi-site pacing, in which electrical stimulation pulses are provided to multiple sites within the same heart chamber. For example, in the electrode configuration shown in FIG. 1, pacing can be provided to the LV using a first pacing channel that includes can electrode 182 as the pacing channel anode and LV electrode 165 as the pacing channel cathode, and using a second pacing channel that includes the can electrode 182 and LV electrode 164. This may be useful to improve coordination of a contraction of the heart chamber, especially contraction of the left ventricle.

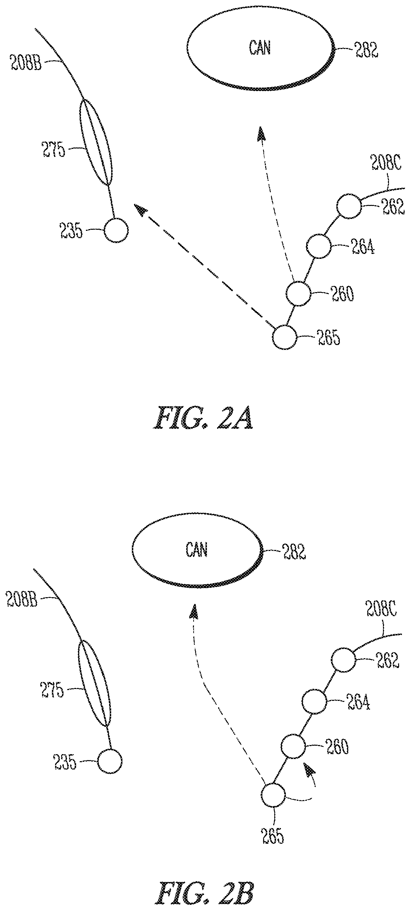

FIGS. 2A and 2B illustrate examples of sensing channels for an ambulatory medical device. Sensing channels can be configured (e.g., by position in the heart chamber) to sense cardiac activity used to detect whether multi-site pacing stimulation energy evoked a response in the cardiac tissue. A sensing channel may include electrodes separate from the electrodes used to deliver the pacing stimulation energy to provide independent pacing and sensing channels. FIG. 2A is an illustration of an example of electrodes configured in independent pacing and sensing channels. The pacing channel includes can electrode 282 as the pacing anode and electrode 260 of the LV lead 208C as the pacing cathode. The sensing channel includes RV coil electrode 275 of the RV lead 208A as the sensing anode and LV electrode 265 as the sensing cathode. One or more of LV electrodes 260, 264, and 262 may be included in additional pacing or sensing channels.

In some variations, the sense channel may include one or more electrodes used to deliver the pacing stimulation energy in a shared cathode sensing configuration. FIG. 2B is an illustration of an example of electrodes configured in a shared cathode sensing configuration. The pacing channel can include LV electrode 260 as the pacing anode and LV electrode 265 as the pacing cathode. The sensing channel can include can electrode 282 as the sensing anode and electrode 265 as the sensing cathode.

Returning to FIG. 1, the example shows that there can be several pacing channels available for multi-site pacing. A clinician may want information on multiple sites to appropriately customize the performance of a device to the specific patient's needs. Medical device based tests can be performed to automatically determine the appropriate electrostimulation energy for pacing therapy delivered using the multiple tissue sites. This can simplify the process of optimizing the device for a specific patient.

However, multi-site pacing can complicate device-based detection of evoked response as part of a cardiac capture test. The mode of the paced contraction may change as stimulation pulse energy is changed so that a response is evoked at both cardiac tissue sites of the heart chamber, at only one cardiac tissue site, or at no cardiac tissue sites. The morphology of sensed cardiac signals can be used to determine which tissue sites induced cardiac capture for the applied electrical stimulus.

Device-based morphology analysis can detect subtle changes in morphology. For instance, the medical device may determine a correlation value between electrogram signals sensed between successive steps of a capture threshold test. An example of a correlation value is feature correlation coefficient (FCC). The FCC can provide an indication of a degree of similarity between the shapes of the electrogram signals. When the FCC value changes to a value greater than a specified FCC threshold value, the morphology between the successive electrogram signals may have changed sufficiently to indicate a shift from multi-site capture to single-site capture. An approach to calculating a correlation value can be found in Kim et al., U.S. Patent No. 7,904,142 filed May 16, 2007. Device-based detection can be easier than visual or manual detection by a clinician and device-based detection can be performed in real time as the stimulation threshold test is performed.

An individual capture test could be performed separately on each electrode separately. However, a cardiac capture test may obtain better results if the test is performed while pacing the heart chamber with multiple electrodes if multi-site pacing is intended. For instance, using multi-site pacing during the capture test may show whether the delay between the multiple sites (if any) is appropriate. For the case of two pacing sites (or dual-site pacing), if the inter-site delay is appropriate, the pacing energy will induce cardiac capture at both pacing sites. If the inter-site delay is not appropriate, the pacing energy will not induce cardiac capture at both pacing sites because, for example, the later tissue site may be in refractory due to the pacing at the first tissue site.

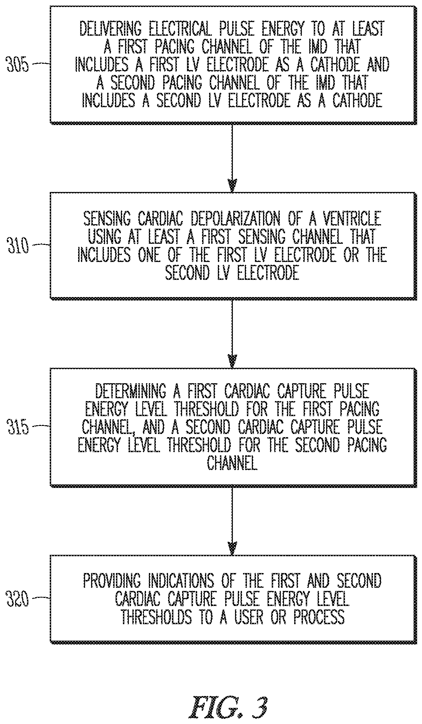

FIG. 3 is a flow diagram of an example of a method 300 of controlling operation of an IMD. At 305, electrical pulse energy is delivered to at least a first pacing channel of the IMD and a second pacing channel of the IMD. The first pacing channel includes a first LV electrode as the cathode of the first pacing channel, and the second pacing channel includes a second LV electrode as the cathode of the second pacing channel. In an illustrative example intended to be non-limiting, the first pacing channel may include the can electrode 182 formed on the implantable housing of FIG. 1 as the pacing anode and LV electrode 165 as the pacing cathode. The second pacing channel may include the can electrode 182 as the pacing anode and LV electrode 160 as the pacing cathode. At 310, cardiac depolarization of the ventricle is sensed using at least a first sensing channel that includes one of the first LV electrode or the second LV electrode. For instance, the sensing channel may include RV coil electrode 175 of FIG. 1 as the sensing anode and LV electrode 160 as the sensing cathode.

At 315, a first cardiac capture pulse energy level threshold is determined for the first pacing channel, and a second cardiac capture pulse energy level threshold for the second pacing channel. The cardiac capture pulse energy level threshold may be determined using a capture test performed by the IMD. At 320, indications of the first and second cardiac capture pulse energy level thresholds are provided to a user or process. An indication of an energy level threshold may include a signal or a value provided to a process executing on the same medical device or a separate medical device. In some variations, the indications are stored in a memory of the IMD and later uploaded to a separate memory device. In some variations, the one or more indications of pacing energy level are provided to a separate medical device for display to a user.

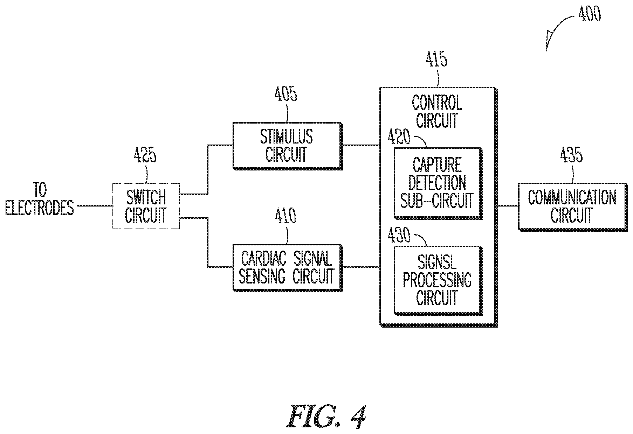

FIG. 4 is a block diagram of portions of an example of a medical device to determine cardiac capture pulse energy level thresholds for multi-site pacing. The device 400 includes a stimulus circuit 405 and a cardiac signal sensing circuit 410 that can be electrically coupled to implantable electrodes. The stimulus circuit 405 provides electrical pulse energy to at least a first pacing channel that includes a first LV electrode as a cathode and a second pacing channel that includes a second LV electrode as a cathode. The cardiac signal sensing circuit 410 senses cardiac activity signals using at least a first sensing channel that includes one of the first LV electrode or the second LV electrode. The device 400 may also include switch circuit 425 to electrically couple different combinations of the electrodes to the stimulus circuit 405 and the cardiac signal sensing circuit 410.

The device 400 also includes a control circuit 415 electrically coupled to the cardiac signal sensing circuit 410 and the stimulus circuit 405. The control circuit 415 can include can include a microprocessor, a digital signal processor, application specific integrated circuit (ASIC), or other type of processor, interpreting or executing instructions included in software or firmware. The control circuit 415 can include sub-circuits to perform the functions described. These sub-circuits may include software, hardware, firmware or any combination thereof. Multiple functions can be performed in one or more of the sub-circuits as desired.

The control circuit 415 includes a capture detection sub-circuit 420 that performs a capture test to determine the cardiac capture pulse energy level thresholds. The capture detection sub-circuit 420 initiates delivery of electrical pulse energy to both the first pacing channel and the second pacing channel and senses cardiac depolarization of a ventricle using the first sensing channel.

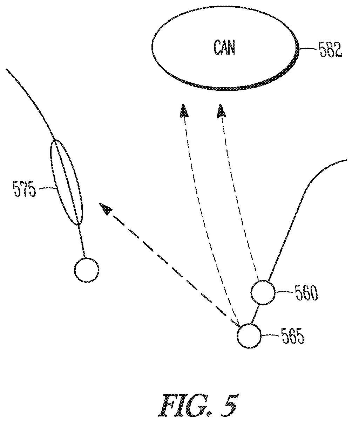

FIG. 5 illustrates portions of an example of a medical device system 500 that includes electrodes and an implantable medical device configured for dual-site pacing. The example shows a first pacing channel that includes LV electrode 565 and can electrode 582, and a second pacing channel that includes LV electrode 560 and can electrode 582. The example also shows a sensing channel that includes LV electrode 565 and RV coil electrode 575. Electrode 565 may be a cathode shared by the first pacing channel and the sensing channel, and the configuration uses one sense channel to sense the evoked response from the electrical pulse energy.

Returning to FIG. 4, the capture detection sub-circuit 420 determines a first cardiac capture pulse energy level threshold for the first pacing channel, and a second cardiac capture pulse energy level threshold for the second pacing channel. The capture detection sub-circuit 420 then provides the first and second cardiac capture pulse energy level thresholds to a user or process.

The electrical pulse energy may be delivered as part of a cardiac capture test performed by the IMD to determine the appropriate pulse energy level thresholds. During the test, the capture detection sub-circuit 420 initiates a change in pulse energy level of the delivered electrical pulse energy. The pacing energy level may be stepped up, stepped down, or step both up and down by the capture detection sub-circuit 420 during the test. One or more cardiac activity signals are sensed in association with a change from a first level of electrical pulse energy to a second level of electrical pulse energy. The sensed cardiac activity signals are then analyzed to determine if there is a change in the evoked response due to the change in pulse energy, such as a change from capture to loss of capture, or from no capture to capture.

A change in the evoked response can be detected by a change in morphology of the signal. When sensing cardiac activity signals using a channel having a cathode shared with a pacing channel, the morphology of the sensed signals exhibits a distinct change when the response changes between no capture (or loss of capture), single-site capture, and dual-site capture. In some examples, the control circuit 415 includes a signal processing sub-circuit 430 that identifies a change in morphology in the sensed cardiac activity signals.

Using the identified change in morphology, the capture detection sub-circuit 420 distinguishes cardiac capture by one of the first and second pacing channels from cardiac capture by both pacing channels. For instance, the dominant peak of the signal may change one or more of polarity, magnitude, and width. The capture detection sub-circuit 420 may also distinguish cardiac capture by one or both of the first and second pacing channels from loss of capture by both pacing channels. For instance the timing of the dominant peak may change when the pacing energy fails to induce an evoked response and the peak in the sensed cardiac activity signal is an intrinsic response occurring later in the cardiac activity signal.

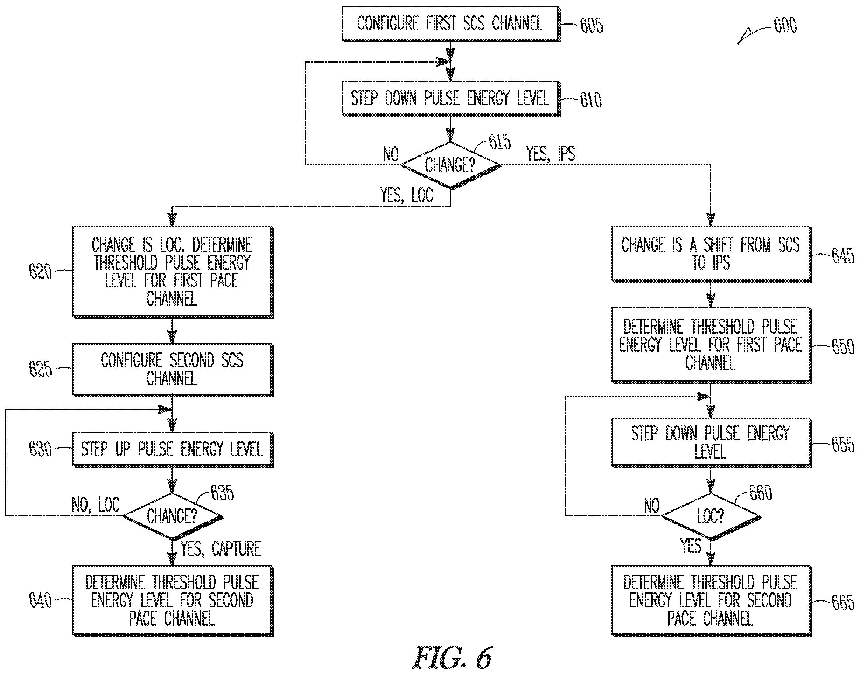

FIG. 6 is a flow diagram of an example of a device-based method 600 of determining pacing capture thresholds for dual-site pacing. The dual-site pacing may include two pacing channels such as the pacing channels shown in the example of FIG. 5, where the first pacing channel includes LV electrode 565 as the cathode and can electrode 582 as the anode, and the second pacing channel includes LV electrode 560 as the cathode and can electrode 582 as the anode.

At 605, an evoked response sensing channel is configured as a shared cathode sensing (SCS) channel. The SCS channel may be configured as shown in FIG. 5 with LV electrode 565 as the shared cathode and either the can electrode 582 or an electrode located in the RV (e.g., RV coil electrode 575) as the sensing channel anode. When the pacing channels and the sensing channel are configured (e.g., by the control circuit 415 of FIG. 4), a capture test is initiated and pulse energy is delivered to the two pacing channels and cardiac activity signals are sensed only with the one shared cathode sensing channel.

At 610, the pulse energy level is changed. In some examples, the pulse energy starts with a high energy level so that both pacing channels induce an evoked response at the cardiac tissue sites, and the pulse energy level is then stepped down at both cardiac tissue sites corresponding to LV electrode 565 and LV electrode 560. At each change in the pulse energy level, one or more cardiac activity signals are sensed with the SCS channel. The device may perform the same change in pulse level more than once during the capture test.

At 615, the stepping of the pulse energy level continues until there is a detected change in signal morphology. The device may look for a change in morphology in a specified time relation to the change in pulse energy level to ensure causality by the pulse energy level change.

Because the test began with capture by both pacing channels, the stepping down of the pulse energy level will eventually result in loss of capture at the first pacing channel or the second pacing channel. If cardiac capture is lost at the second pacing channel first, the SCS channel will still detect capture because the cathode is shared with the first pacing channel. Further stepping down of the pulse energy level will result in loss of capture by both pacing channels and the sensed signal morphology will show total loss of capture. If cardiac capture is first lost at the first pacing channel, the sensed signal morphology will appear to be sensing associated with independent pacing and sensing (IPS) channels because capture continues at the second pacing channel which does not share a cathode with the sensing channel. When a change in the sensed signals is detected, the capture detection sub-circuit 420 identifies the detected change in signal morphology.

If the change in morphology corresponds to a shift from a morphology indicating cardiac capture sensed by a shared cathode sensing channel to a morphology indicating loss of capture (LOC) by the pacing channel, the method 600 follows the left branch of the flow diagram to 620. The capture detection sub-circuit 420 may identify the change as a change to LOC morphology by a shift in time of the dominant peak of the sensed cardiac activity signal. The shift in time may occur because the new dominant peak is associated with an intrinsic response that occurs later than the pace pulse.

At 620, the threshold pulse energy level is determined for the first pacing channel. For instance, the pulse energy level just prior to that which resulted in loss of capture is recorded. A safety margin may then be added to the recorded pulse energy level to set the threshold pulse energy level.

At 625, the cardiac signal sensing circuit 410 is configured to sense cardiac activity signals using only a second sensing channel. The second sensing may also be a SCS channel that shares a cathode with the second pacing channel.

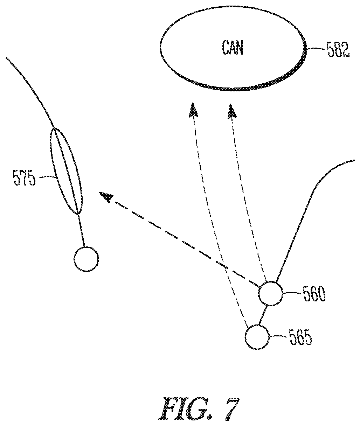

FIG. 7 shows the example of FIG. 5 with a second shared cathode sensing channel comprising RV coil electrode 575 as the sensing node and LV electrode 560 as the shared cathode. In certain examples, the first shared cathode sensing channel is disabled and sensing continues with the second shared cathode sensing channel. In certain examples, the first shared cathode sensing channel is reconfigured by the control circuit 415 to share the cathode with the second pacing channel.

Returning to FIG. 6, subsequent cardiac activity signals are then sensed using the second shared cathode sensing channel. The change in the signal morphology may have occurred because capture was first lost by the second pacing first, or both the first and second pacing channels lost capture by the same change in pulse energy.

At 630, the pulse energy level is stepped up to find the threshold for the second pacing channel. At 635, capture is detected when the morphology of the sensed cardiac activity signals changes from LOC to capture sensed with SCS by the second sensing channel. The pulse energy level that resulted in capture by the second pacing channel is recorded at 640. The pulse energy level threshold for the second pacing channel may be determined by adding a safety margin to the recorded pulse energy level.

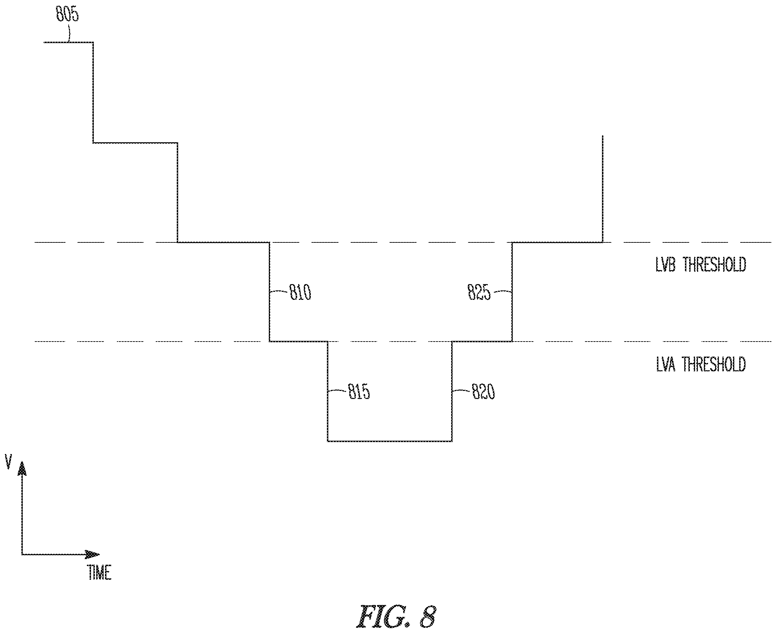

FIG. 8 is a graph that illustrates the threshold determination in the left branch of the method 600 of FIG. 6. In the graph, the vertical direction represents pulse energy and the horizontal direction represents time. At the top left of the graph, evoked response (ER) in the cardiac activity signals is sensed using the first shared cathode sensing channel. At 805, cardiac capture by both pacing channels is evident in the sensed cardiac activity signals and has a morphology corresponding to cardiac capture sensed by a shared cathode sensing channel. Moving to the right in the graph, the pulse energy level is stepped down.

At 810, cardiac capture is lost by the second pacing channel (e.g., LV electrode 560 in FIG. 5). The pulse energy level continues to be stepped down. At 815, cardiac capture is lost by the first pacing channel (e.g., LV electrode 565 in FIG. 5) and neither pacing channel captures the heart. The LOC may be detected as a shift to a signal morphology that is associated with complete loss of capture. The pulse energy level just prior to loss of capture by the first pacing channel is recorded. The sensing of the cardiac activity signals is changed over to the second shared cathode sensing channel. There is no evidence of capture by either pacing channel in cardiac activity signals sensed with the second shared cathode sensing channel at this point. The pulse energy level is stepped up.

At 820, capture by the first pacing channel may be evident in cardiac activity signals sensed with the second shared cathode sensing channel. The morphology would be associated with IPS because the second sensing channel does not share a cathode with the first pacing channel. At 825, cardiac capture by both the first and second pacing channels is evident in cardiac activity signals sensed with the second shared cathode sensing channel. The pulse energy level that induced cardiac capture by the second pacing channel is recorded.

Returning to FIG. 6, as explained previously, a change in morphology is detected at 615 and the capture detection sub-circuit 420 identifies the change. At this point in the flow diagram, the shared cathode sensing channel still shares the cathode with the first pacing channel (e.g., LV electrode 565 in FIG. 5). If the change in morphology corresponds to a change from a morphology indicating capture sensed using a SCS channel to a morphology indicating capture sensed using an IPS channel, the method 600 branches to the right at 645 where the capture detection sub-circuit 420 identifies the change as SCS to IPS.

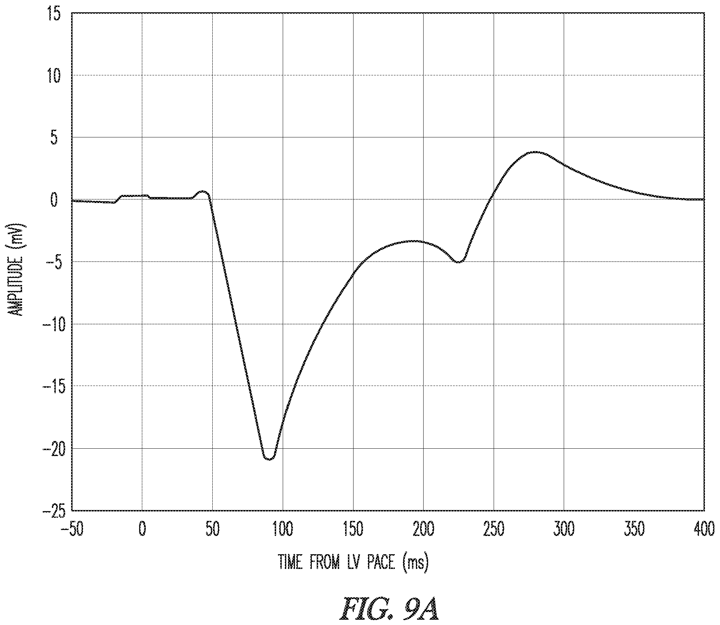

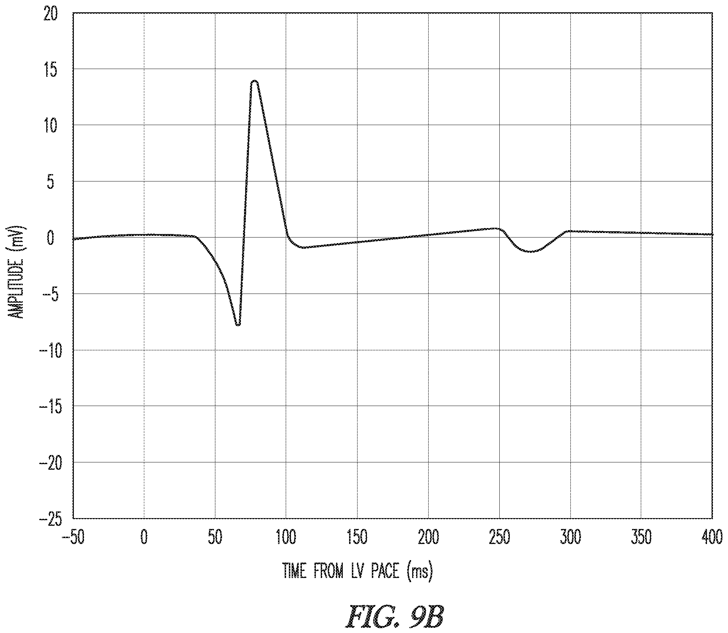

FIGS. 9A and 9B show an example of a shift in sensed cardiac signal morphology from SCS to IPS. FIG. 9A shows an example of morphology of capture sensed with SCS. The example shows one dominant peak in the morphology and in the example the peak is negative in amplitude. FIG. 9B shows an example of morphology of capture sensed with IPS. The example shows a bimodal morphology with a dominant positive peak. Thus, in the examples of FIGS. 9A and 9B, the capture detection sub-circuit 420 identifies the change from SCS to IPS based on the detected shift in morphology from FIG. 9A to FIG. 9B.

Returning to FIG. 6 at 650, the pulse energy level threshold just prior to that which resulted in the shift from SCS morphology to IPS morphology is recorded (e.g., stored in device memory) for the first pacing channel. A safety margin may be added to the recorded energy level to set the pulse energy threshold for the first pacing channel. At 655, the step down of the pulse energy level continues until the sensed cardiac activity signals indicate LOC. The capture detection sub-circuit 420 may identify the change from IPS to LOC at 660 from the shift in signal morphology. The pulse energy level just prior to that which resulted in the shift to LOC is recorded for the second pacing channel. Note that the shared cathode sensing channel in the right branch of the method of FIG. 6 is not changed as it was for the left branch of FIG. 6. At 665, the pulse energy level is determined using the recorded pulse energy level.

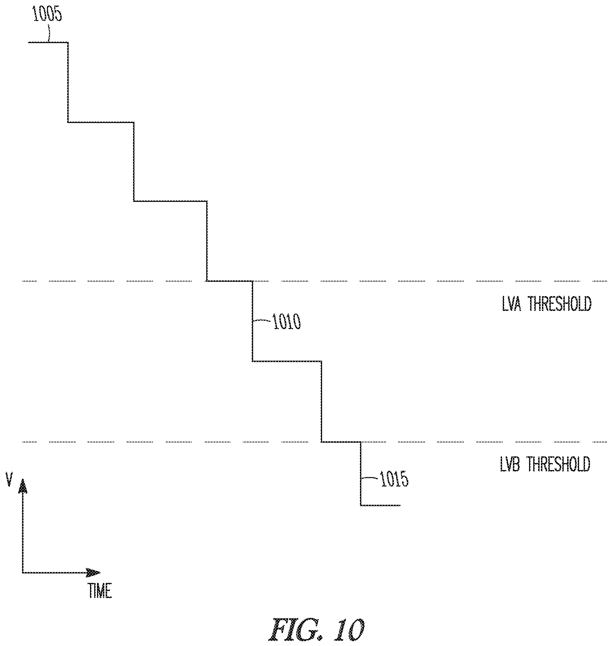

FIG. 10 is a graph that illustrates the threshold determination in the right branch of the method of FIG. 6. As in the graph of FIG. 8, the vertical direction represents pulse energy and the horizontal direction represents time. At the top left of the graph, the cardiac activity signals are sensed using the first shared cathode sensing channel (e.g., LV electrode 565 in FIG. 5 is shared by the first sensing channel and the first pacing channel). At 1005, cardiac capture by both pacing channels is evident in the sensed cardiac activity signals and has a signal morphology indicating cardiac capture sensed by a SCS channel. Moving to the right in the graph, the pulse energy level is stepped down.

At 1010, cardiac capture is lost by the first pacing channel and the morphology of sensed cardiac signal changes from a morphology indicating capture sensed using SCS to a morphology indicating capture sensed using IPS. The second pacing channel (e.g., a pacing channel with LV electrode 560 in FIG. 5) continues to capture the heart. The pulse energy level delivered just prior to the pulse energy level that resulted in the morphology shift is recorded and is used to determine the pulse energy threshold for the first pacing channel.

At 1015, cardiac capture is lost by the second pacing channel, and the morphology of sensed cardiac activity signals change from a morphology indicating capture sensed using IPS to a morphology indicating LOC. The pulse energy level delivered to the pulse energy level that resulted in LOC is recorded and is used to determine the pulse energy threshold for the second pacing channel.

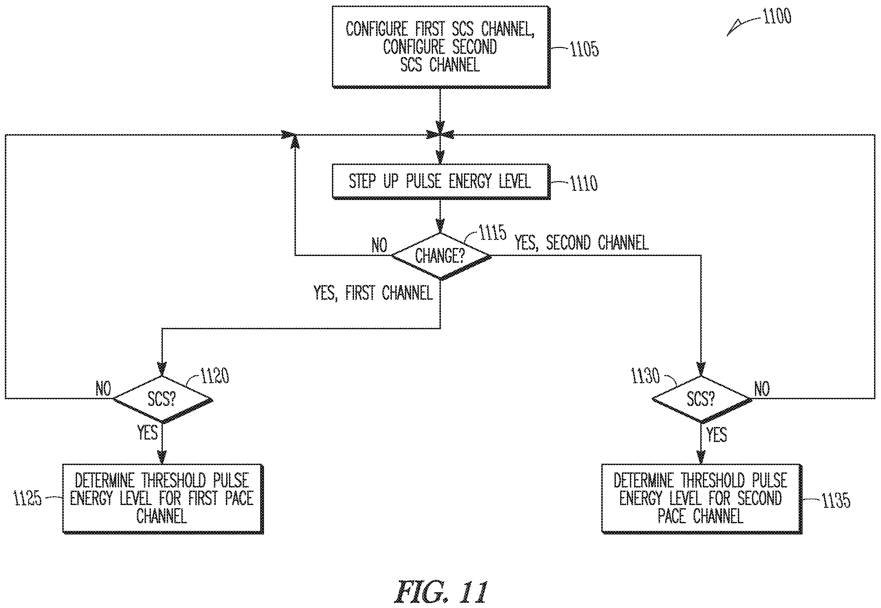

FIG. 11 is a flow diagram of another example of a method 1100 of determining pacing capture thresholds for dual-site pacing. The dual-site pacing may include two pacing channels and two sensing channels. For dual-site pacing in the LV, the stimulus circuit 405 of FIG. 4 provides pulse energy to a first pacing channel that includes a first LV electrode (e.g., LV electrode 565 in FIG. 5) and the cardiac signal sensing circuit 410 senses cardiac activity signals using a first sensing channel that includes the first LV electrode as a sensing cathode. The stimulus circuit 405 also provides pulse energy to a second pacing channel that includes a second LV electrode (e.g., LV electrode 560 in FIG. 5) and the cardiac signal sensing circuit 410 senses cardiac activity signals using a second sensing channel that includes the first LV electrode as a sensing cathode. Thus, each of the sensing channels is configured to share a cathode with one of the pacing channels. This is shown in FIG. 11 at 1105.

At 1110, the capture detection sub-circuit 420 initiates a change in pulse energy level provided to the first and second pacing channels. In the example of FIG. 11, the pulse energy level is stepped up from a pulse energy level at which cardiac capture (by either pacing channel) is not evident in sensed cardiac activity signals. Alternatively, the capture detection sub-circuit 420 may initiate the capture test with a pulse energy level that ensures cardiac capture by both pacing channels and the pulse energy level is then stepped down. Cardiac activity signals are sensed using both SCS channels after the pulse energy level is changed.

At 1115, the capture detection sub-circuit 420 uses the sensed signals to detect a change in the mode of cardiac capture. Assuming a step up test, the capture detection sub-circuit 420 uses the sensed signals to detect a change from absence of capture to capture by one or both of the pacing channels. If the pulse energy was stepped down, the capture detection sub-circuit 420 check for a change from capture to loss of capture.

At 1120, the capture detection sub-circuit 420 checks whether a cardiac activity signal sensed using the first SCS channel displays a capture morphology associated with a shared cathode sensing. If the sensed signal does display a capture morphology, the pulse energy level associated with the detected change is recorded. At 1125, the cardiac capture pulse energy level threshold is determined for the first pacing channel using the recorded pulse energy level. In some examples, a safety margin is added to the recorded pulse energy level to determine the cardiac capture pulse energy level threshold. If the sensed signal does not display a capture morphology, the method returns to 1110 to continue stepping the pulse energy level.

At 1130, the capture detection sub-circuit 420 checks whether a cardiac activity signal sensed using the second SCS channel displays a capture morphology associated with a shared cathode sensing. If the sensed signal does display a capture morphology, the pulse energy level associated with the detected change is recorded.

At 1135, the cardiac capture pulse energy level threshold is determined for the first pacing channel. If the sensed signal does not display a morphology associated with cardiac capture, the method returns to 1110 to continue stepping the pulse energy level. The energy level is stepped until cardiac capture by both pacing channels is detected.

The methods in the examples of FIGS. 6 and 11 can be expanded from dual-site pacing to include more than two pacing sites. For instance, after the method is completed for two heart chamber electrodes (e.g., LV electrodes 265 and 260 in FIG. 2A), the capture test can be run using another pair of electrodes (e.g., LV electrodes 264 and 262 in FIG. 2A) as pacing channels. Sensing channels can be configured to share the sensing cathode with a pacing channel cathode. For tri-site pacing, two pairs of electrodes can be selected with one electrode common to the pairs. The method can be performed for the first pair and then the second.

For more than quad-site pacing, pairs of the electrodes can be selected for pacing channels. A pair of pacing channels is selected for determining pace pulse energy thresholds. One or more cardiac signal sensing channel is selected that includes an LV electrode from the selected pair of pacing channels. Cardiac capture pulse energy level thresholds are determined for the selected pacing channels, pairs of the pacing channels continue to be selected and cardiac capture pulse energy level thresholds can be determined for the selected pairs until a cardiac capture pulse energy level threshold is determined for each of the pacing channels.

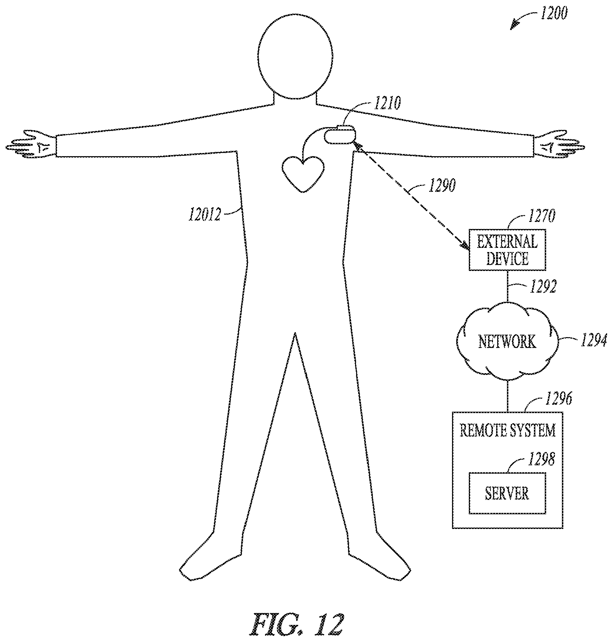

FIG. 12 is an illustration of portions of another system 1200 that uses an IMD 1210 to provide a therapy to a patient 1202. The system 1200 typically includes an external device 1270 that communicates signals 1290 wirelessly with a remote system 1296 via a network 1294. The network 1294 can be a communication network such as a phone network or a computer network (e.g., the internet). In some examples, the external device 1270 includes a repeater and communicated via the network using a link 1292 that may be wired or wireless. In some examples, the remote system 1296 provides patient management functions and may include one or more servers 1298 to perform the functions.

Returning to FIG. 4, the device 400 can include a communication circuit 435 to communicate information with a separate device, such as the external device 1270 of FIG. 12. When the cardiac capture pulse energy level thresholds are determined for the pacing channels by the capture detection sub-circuit 420, the control circuit 415 communicates indications of the cardiac capture pulse energy level thresholds to the separate device. In some variations, the indications may be stored in a memory of the device 400 and later uploaded by the separate device. In some variations, the indications could be communicated to the separate device in real time as the tests are performed. When the indications are communicated to the separate device they may be displayed to a user.

In some examples, the device 400 provides pacing therapy to the subject using the stimulus circuit 405 and the device updates the pacing thresholds for subsequent pacing therapy with the determined cardiac capture pulse energy level thresholds. In some examples, the pacing thresholds are updated after a confirmation from the separate device is received. Because the pacing threshold values are optimized by the capture test, the updated pacing parameters provide effective therapy to the subject while optimizing battery life of the device.

Assistance by the CRM device in determining pacing thresholds can be especially beneficial if the clinician would like information concerning a large number or all of the possible pacing channels than can be configured by the device using deployed electrodes. The optimum stimulation threshold may vary over time for a patient due to maturation of myocardial tissue around an implanted electrode, drug therapy prescribed to the patient, an episode of myocardial infarction, and defibrillation of the myocardial tissue. Therefore, the tests may be run more than once by a device while the device is implanted.

The devices and methods described herein allow for multi-site stimulation to be delivered to the subject with the minimum stimulation energy required for effective therapy. The device may provide additional information related to the multi-site pacing to assist the clinician or caregiver in managing the device-based therapy.

The above detailed description includes references to the accompanying drawings, which form a part of the detailed description. The drawings show, by way of illustration, specific embodiments in which the invention can be practiced. These embodiments are also referred to herein as "examples."

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times. These computer-readable media can include, but are not limited to, hard disks, removable magnetic disks, removable optical disks (e.g., compact disks and digital video disks), magnetic cassettes, memory cards or sticks, random access memories (RAM's), read only memories (ROM's), and the like. In some examples, a carrier medium can carry code implementing the methods. The term "carrier medium" can be used to represent carrier waves on which code is transmitted.

The above description is intended to be illustrative, and not restrictive. The scope of the invention should be determined with reference to the appended claims.

## Claims

1. An apparatus for electrical coupling to a plurality of implantable electrodes, the apparatus comprising:
a stimulus circuit (405) configured to provide electrical pulse energy to at least a first pacing channel that includes a first left ventricular (LV) electrode (165; 260; 565) as a cathode and a second pacing channel that includes a second LV electrode (164; 262, 264; 560) as a cathode;
a cardiac signal sensing circuit (410) configured to sense cardiac activity signals using at least a first sensing channel that includes one of the first LV electrode (165; 260; 565) or the second LV electrode (164; 262, 264; 560);
a control circuit (415) electrically coupled to the cardiac signal sensing circuit (410) and the stimulus circuit (405), wherein the control circuit (415) includes a capture detection sub-circuit (420) configured to: initiate delivery of electrical pulse energy to both the first pacing channel and the second pacing channel; sense cardiac depolarization of a ventricle using the first sensing channel; determine a first cardiac capture pulse energy level threshold for the first pacing channel, and a second cardiac capture pulse energy level threshold for the second pacing channel; and provide indications of the first and second cardiac capture pulse energy level thresholds to a user or process,
wherein the capture detection sub-circuit (420) is configured to initiate a change in pulse energy level of the electrical pulse energy delivered to both the first pacing channel and the second pacing channel;
wherein the control circuit (415) includes a signal processing sub-circuit (430) configured to identify a change in morphology in a cardiac activity signal sensed in association with a change from a first level of electrical pulse energy delivered to both the first pacing channel and the second pacing channel to a second level of electrical pulse energy delivered to both the first pacing channel and the second pacing channel; and
wherein the capture detection sub-circuit (420) is further configured to distinguish, using the identified change in morphology, between cardiac capture by one of the first and second pacing channels from cardiac capture by both pacing channels.

2. The apparatus of claim 1, wherein the capture detection sub-circuit (420) is configured to distinguish, using the identified change in morphology, between cardiac capture by one or both of the first and second pacing channels from loss of capture by both pacing channels.

3. The apparatus of claim 1, wherein the capture detection sub-circuit (420) is configured to identify a change from capture by a pacing channel to loss of capture by the pacing channel when a cardiac activity signal sensed using a sensing channel that shares an electrode (265; 560, 565) with the pacing channel indicates a change from a signal morphology indicating capture sensed using a cathode shared with a pacing channel to a signal morphology indicating absence of cardiac capture.

4. The apparatus of claim 1, wherein the capture detection sub-circuit (420) is configured to identify a change from capture by both of the first and second pacing channels to capture by one pacing channel when a cardiac activity signal sensed using a sensing channel that shares an electrode (265; 560, 565) with the pacing channel indicates a change from a signal morphology indicating capture sensed using a cathode shared with a pacing channel to a signal morphology indicating capture sensed using a cathode independent from a pacing channel.

5. The apparatus of claim 1, wherein the control circuit (415) includes a signal processing sub-circuit (430) configured to identify a change in morphology in a sensed cardiac activity signal that indicates shared cathode sensing by a signal sensing channel and occurs in a time relation to the change in pulse energy level; and wherein the capture detection sub-circuit (420) is configured to identify a change from absence of capture by a pacing channel to capture by the pacing channel when the change in morphology is identified.

6. The apparatus of claim 1,
wherein the first sensing channel includes the first LV electrode (165; 260; 565) as a sensing cathode,
wherein the cardiac signal sensing circuit (410) is further configured to sense cardiac activity signals using a second sensing channel that includes the second LV electrode (164; 262, 264; 560) as a sensing cathode,
wherein the capture detection sub-circuit (420) is configured to:
monitor cardiac activity signals using only the first sensing channel;
detect loss of capture by the first pacing channel using the first sensing channel;
determine a first cardiac capture pulse energy level threshold for the first pacing channel using a pulse energy level associated with the loss of capture by the first pacing channel;
change to monitoring cardiac activity signals using only the second sensing channel after the loss of capture by the first pacing channel is detected;
detect loss of capture by the second pacing channel using the second sensing channel; and
determine a second cardiac capture pulse energy level threshold for the second pacing channel using a pulse energy level associated with the loss of capture by the second pacing channel.

7. The apparatus of claim 1,
wherein the first sensing channel includes the first LV electrode (165; 260; 565) as a sensing cathode and is configured to sense a first cardiac activity signal;
wherein the cardiac signal sensing circuit (410) is further configured to sense a second cardiac activity signal using a second sensing channel that includes the second LV electrode (164; 262, 264; 560) as a sensing cathode,
wherein the capture detection sub-circuit (420) is configured to:
detect a change between capture and loss of capture by the first pacing channel using the first cardiac activity channel and determine the first cardiac capture pulse energy level threshold for the first pacing channel using a pulse energy level associated with the detected change; and
detect a change between capture and loss of capture by the second pacing channel using the second cardiac activity channel and determine the second cardiac capture pulse energy level threshold for the second pacing channel using a pulse energy level associated with the detected change.

8. The apparatus of claim 1, including an implantable housing (150; 282; 582), wherein the first sensing channel includes the first LV electrode (165; 260; 565) as the sensing cathode and an electrode formed on the implantable housing (150; 282; 582) as the sensing anode.

9. The apparatus of claim 1, wherein the cardiac signal sensing circuit (410) is configured to be electrically coupled to a right ventricular (RV) electrode, and wherein the first sensing channel includes the first LV electrode (165; 260; 565) as the sensing cathode and the RV electrode (175; 275; 575) as the sensing anode.

10. The apparatus of one or any combination of claims 1-9, wherein the stimulus circuit (405) is configured to provide the electrical pulse energy to more than two pacing channels, wherein each pacing channel includes a different LV electrode (164, 165; 260, 262, 264; 560, 565), and wherein the capture detection sub-circuit (420) is configured to:
select a pair of the pacing channels;
select a cardiac signal sensing channel that includes an LV electrode (164, 165; 260, 262, 264; 560, 565) from the selected pair of pacing channels;
determine cardiac capture pulse energy level thresholds for the selected pacing channels;
and
continue to select pairs of the pacing channels and determine cardiac capture pulse energy level thresholds for the selected pairs until a cardiac capture pulse energy level threshold is determined for each of the pacing channels.

11. A machine-readable medium including instructions that, when performed by the apparatus of any of claims 1-10, cause the apparatus to perform acts comprising:
delivering electrical pulse energy to both of at least a first pacing channel of the apparatus that includes a first left ventricular (LV) electrode (165; 260; 565) as a cathode and a second pacing channel of the apparatus
that includes a second LV electrode (164; 262, 264; 560) as a cathode;
sensing cardiac depolarization of a ventricle using at least a first sensing channel that includes one of the first LV electrode (165; 260; 565) or the second LV electrode (164; 262, 264; 560);
changing a pulse energy level of the electrical pulse energy delivered to both of the first and second pacing channel;
identifying a change in morphology in a cardiac activity signal sensed in association with a change from a first level of electrical pulse energy delivered to both the first pacing channel and the second pacing channel to a second level of electrical pulse energy delivered to both the first pacing channel and the second pacing channel;
distinguishing, using the identified change in morphology, cardiac capture by one of the first and second pacing channels from cardiac capture by both pacing channels;
determining a first cardiac capture pulse energy level threshold for the first pacing channel, and a second cardiac capture pulse energy level threshold for the second pacing channel; and
providing indications of the first and second cardiac capture pulse energy level thresholds to a user or process.

12. The machine readable medium of claim 11 including instructions that, when performed by the apparatus, cause the apparatus to: distinguish, using the identified change in morphology, cardiac capture by one or both of the first and second pacing channels from loss of capture by both pacing channels.

13. The machine readable medium of claim 11 or 12, including instructions that, when performed by apparatus, cause the apparatus to: identify a change from capture by a pacing channel to loss of capture by the pacing channel when a cardiac activity signal sensed using a sensing channel that shares an electrode with the pacing channel indicates a change from a signal morphology indicating capture sensed using a cathode shared with a pacing channel to a signal morphology indicating absence of cardiac capture.

## Patentansprüche

1. Vorrichtung zur elektrischen Kopplung an mehrere implantierbare Elektroden, wobei die Vorrichtung umfasst:
einen Schaltkreis zur Stimulation (405), dafür ausgelegt, elektrische Impulsenergie bereitzustellen an mindestens einen ersten Stimulationskanal, der eine erste linksventrikuläre (LV) Elektrode (165; 260; 565) als Kathode umfasst, und einen zweiten Stimulationskanal, der eine zweite LV-Elektrode (164; 262, 264; 560) als Kathode umfasst;
einen Schaltkreis zum Erfassen kardialer Signale (410), dafür ausgelegt, die Signale einer Herzaktivität zu erfassen, unter Verwendung von mindestens einem ersten Sensing-Kanal, der entweder die erste LV-Elektrode (165; 260; 565) oder die zweite LV-Elektrode (164; 262, 264; 560) umfasst;
einen Steuerschaltkreis (415), der elektrisch mit dem Schaltkreis zum Erfassen kardialer Signale (410) und dem Schaltkreis zur Stimulation (405) gekoppelt ist, wobei der Steuerschaltkreis (415) einen Teilschaltkreis zur Capture-Detektion (420) umfasst, dafür ausgelegt: die Bereitstellung elektrischer Impulsenergie an den ersten Stimulationskanal wie auch den zweiten Stimulationskanal einzuleiten; ein Erfassen einer kardialen Depolarisation eines Ventrikels unter Verwendung des ersten Sensing-Kanals vorzunehmen; für den ersten Stimulationskanal eine erste kardiale Capture-Impulsenergieniveau-Schwelle und für den zweiten Stimulationskanal eine zweite kardiale Capture-Impulsenergieniveau-Schwelle zu bestimmen; und an einen Benutzer oder Prozess Angaben zur ersten und zweiten kardialen Capture-Impulsenergieniveau-Schwelle auszugeben,
wobei der Teilschaltkreis zur Capture-Detektion (420) dafür ausgelegt ist, eine Änderung in Bezug auf das Impulsenergieniveau der elektrischen Impulsenergie, die an den ersten Stimulationskanal wie auch den zweiten Stimulationskanal bereitgestellt wird, einzuleiten;
wobei der Steuerschaltkreis (415) einen Teilschaltkreis zur Signalverarbeitung (430) umfasst, dafür ausgelegt, eine Änderung in der Morphologie eines Herzaktivitätssignals zu erkennen, die in Verbindung mit einer Änderung von einem ersten Niveau der elektrischen Impulsenergie, die an den ersten Stimulationskanal wie auch den zweiten Stimulationskanal bereitgestellt wird, hin zu einem zweiten Niveau der elektrischen Impulsenergie, die an den ersten Stimulationskanal wie auch den zweiten Stimulationskanal bereitgestellt wird, erfasst wird;
und
wobei der Teilschaltkreis zur Capture-Detektion (420) ferner dafür ausgelegt ist, unter Verwendung der erkannten Änderung in der Morphologie, zwischen einem Capture-Vorgang durch entweder den ersten oder den zweiten Stimulationskanal und einem Capture-Vorgang durch beide Stimulationskanäle zu unterscheiden.

2. Vorrichtung nach Anspruch 1, wobei der Teilschaltkreis zur Capture-Detektion (420) dafür ausgelegt ist, unter Verwendung der erkannten Änderung in der Morphologie, zwischen einem Capture-Vorgang durch einen oder beide Stimulationskanäle und einem Capture-Verlust durch beide Stimulationskanäle zu unterscheiden.

3. Vorrichtung nach Anspruch 1, wobei der Teilschaltkreis zur Capture-Detektion (420) dafür ausgelegt ist, eine Änderung von einem Capture-Vorgang durch einen Stimulationskanal hin zu einem Capture-Verlust durch den Stimulationskanal zu erkennen, wenn ein Herzaktivitätssignal, das unter Verwendung eines Sensing-Kanals, der eine Elektrode (265; 560, 565) mit dem Stimulationskanal teilt, erfasst worden ist, auf eine Änderung von einer Signalmorphologie, die auf ein Capture-Sensing durch eine mit einem Stimulationskanal geteilte Kathode hindeutet, hin zu einer Signalmorphologie, die auf die Abwesenheit eines Capture-Vorgangs hindeutet, hinweist.

4. Vorrichtung nach Anspruch 1, wobei der Teilschaltkreis zur Capture-Detektion (420) dafür ausgelegt ist, eine Änderung von einem Capture-Vorgang durch den ersten wie auch den zweiten Stimulationskanal hin zu einem Capture-Vorgang durch nur einen Stimulationskanal zu erkennen, wenn ein Herzaktivitätssignal, das unter Verwendung eines Sensing-Kanals, der eine Elektrode (265; 560, 565) mit dem Stimulationskanal teilt, erfasst worden ist, auf eine Änderung von einer Signalmorphologie, die auf ein Capture-Sensing durch eine mit einem Stimulationskanal geteilte Kathode hindeutet, hin zu einer Signalmorphologie, die auf ein Capture-Sensing durch eine von einem Stimulationskanal unabhängige hindeutet, hinweist.

5. Vorrichtung nach Anspruch 1, wobei der Steuerschaltkreis (415) einen Teilschaltkreis zur Signalverarbeitung (430) umfasst, der dafür ausgelegt ist, eine Änderung in der Morphologie eines erfassten Herzaktivitätssignals zu erkennen, die auf ein Erfassen mittels geteilter Kathode durch einen Signal-Sensing-Kanal hinweist und in einer zeitlichen Beziehung zur Änderung des Impulsenergieniveaus geschieht; und wobei der Teilschaltkreis zur Capture-Detektion (420) dafür ausgelegt ist, eine Änderung von Abwesenheit eines Capture-Vorgangs durch einen Stimulationskanal hin zu einem Capture-Vorgang durch den Stimulationskanal zu erkennen, wenn die Änderung in der Morphologie erkannt wird.

6. Vorrichtung nach Anspruch 1,
wobei der erste Sensing-Kanal die erste LV-Elektrode (165; 260; 565) als Sensing-Kathode umfasst,
wobei der Schaltkreis zum Erfassen kardialer Signale (410) ferner dafür ausgelegt ist, Herzaktivitätssignale unter Verwendung eines zweiten Sensing-Kanals zu erfassen, der die zweite LV-Elektrode (164; 262, 264; 560) als Sensing-Kathode umfasst,
wobei der Teilschaltkreis zur Capture-Detektion (420) dafür ausgelegt ist:
die Herzaktivitätssignale unter Verwendung nur des ersten Sensing-Kanals zu überwachen;
Capture-Verlust durch den ersten Stimulationskanal unter Verwendung des ersten Sensing-Kanals zu detektieren;
für den ersten Stimulationskanal eine erste kardiale Capture-Impulsenergieniveau-Schwelle unter Verwendung eines Impulsenergieniveaus, das mit dem Capture-Verlust durch den ersten Stimulationskanal verknüpft ist, zu bestimmen;
zu einer Überwachung der Herzaktivitätssignale unter Verwendung nur des zweiten Sensing-Kanals zu wechseln, nachdem der Capture-Verlust durch den ersten Stimulationskanal detektiert worden ist;
Capture-Verlust durch den zweiten Stimulationskanal unter Verwendung des zweiten Sensing-Kanals zu detektieren; und
für den zweiten Stimulationskanal eine zweite kardiale Capture-Impulsenergieniveau-Schwelle unter Verwendung eines Impulsenergieniveaus, das mit dem Capture-Verlust durch den zweiten Stimulationskanal verknüpft ist, zu bestimmen.

7. Vorrichtung nach Anspruch 1,
wobei der erste Sensing-Kanal die erste LV-Elektrode (165; 260; 565) als Sensing-Kathode umfasst und dafür ausgelegt ist, ein erstes Herzaktivitätssignal zu erfassen;
wobei der Schaltkreis zum Erfassen kardialer Signale (410) ferner dafür ausgelegt ist, ein zweites Herzaktivitätssignal unter Verwendung eines zweiten Sensing-Kanals zu erfassen, der die zweite LV-Elektrode (164; 262, 264; 560) als Sensing-Kathode umfasst,
wobei der Teilschaltkreis zur Capture-Detektion (420) dafür ausgelegt ist:
eine Änderung zwischen Capture-Vorgang und Capture-Verlust durch den ersten Stimulationskanal unter Verwendung des ersten Herzaktivitätskanals zu detektieren und für den ersten Stimulationskanal die erste kardiale Capture-Impulsenergieniveau-Schwelle unter Verwendung eines Impulsenergieniveaus, das mit der detektierten Änderung verknüpft ist, zu bestimmen; und
eine Änderung zwischen Capture-Vorgang und Capture-Verlust durch den zweiten Stimulationskanal unter Verwendung des zweiten Herzaktivitätskanals zu detektieren und für den zweiten Stimulationskanal die zweite kardiale Capture-Impulsenergieniveau-Schwelle unter Verwendung eines Impulsenergieniveaus, das mit der detektierten Änderung verknüpft ist, zu bestimmen.

8. Vorrichtung nach Anspruch 1, ein implantierbares Gehäuse (150; 282; 582) umfassend, wobei der erste Sensing-Kanal die erste LV-Elektrode (165; 260; 565) als die Sensing-Kathode und eine Elektrode, die am implantierbaren Gehäuse (150; 282; 582) ausgebildet ist, als die Sensing-Anode umfasst.

9. Vorrichtung nach Anspruch 1, wobei der Schaltkreis zum Erfassen kardialer Signale (410) dafür ausgelegt ist, elektrisch mit einer rechtsventrikulären (RV) Elektrode gekoppelt zu werden, und wobei der ersten Sensing-Kanal die erste LV-Elektrode (165; 260; 565) als Sensing-Kathode und die RV-Elektrode (175; 275; 575) als Sensing-Anode umfasst.

10. Vorrichtung nach einem der Ansprüche 1 bis 9 oder jeder Kombination daraus, wobei der Schaltkreis zur Stimulation (405) dafür ausgelegt ist, die elektrische Impulsenergie an mehr als zwei Stimulationskanäle bereitzustellen, wobei jeder Stimulationskanal eine andere LV-Elektrode (164, 165; 260, 262, 264; 560, 565) umfasst, und wobei der Teilschaltkreis zur Capture-Detektion (420) dafür ausgelegt ist:
ein Stimulationskanal-Paar auszuwählen;
einen Sensing-Kanal für kardiale Signale, der eine LV-Elektrode (164, 165; 260, 262, 264; 560, 565) umfasst, aus dem ausgewählten Stimulationskanal-Paar auszuwählen;
kardiale Capture-Impulsenergieniveau-Schwellen für die ausgewählten Stimulationskanäle zu bestimmen; und
fortzufahren, Stimulationskanal-Paare auszuwählen und kardiale Capture-Impulsenergieniveau-Schwellen für die ausgewählten Paare zu bestimmen, bis für jeden der Stimulationskanäle eine kardiale Capture-Impulsenergieniveau-Schwelle bestimmt ist.

11. Maschinenlesbares Medium, einschließlich Anweisungen, die, wenn sie durch die Vorrichtung nach einem der Ansprüche 1 bis 10 ausgeführt werden, bewirken, dass die Vorrichtung Vorgänge ausführt, umfassend:
Bereitstellung elektrischer Impulsenergie an beide von mindestens einem ersten Stimulationskanal der Vorrichtung, der eine erste linksventrikuläre (LV) Elektrode (165; 260; 565) als Kathode umfasst, und einem zweiten Stimulationskanal der Vorrichtung, der eine zweite LV-Elektrode (164; 262, 264; 560) als Kathode umfasst;
Erfassen einer kardialen Depolarisation eines Ventrikels, unter Verwendung von mindestens einem ersten Sensing-Kanal, der entweder die erste LV-Elektrode (165; 260; 565) oder die zweite LV-Elektrode (164; 262, 264; 560) umfasst;
Änderung des Energieimpulsniveaus der elektrischen Impulsenergie, die an den ersten wie auch den zweiten Stimulationskanal bereitgestellt wird;
Erkennung einer Änderung in der Morphologie eines Herzaktivitätssignals, das in Verbindung mit einer Änderung von einem ersten Niveau einer elektrischen Impulsenergie, die an den ersten Stimulationskanal wie auch den zweiten Stimulationskanal bereitgestellt wird, hin zu einem zweiten Niveau einer elektrischen Impulsenergie, die an den ersten Stimulationskanal wie auch den zweiten Stimulationskanal bereitgestellt wird, erfasst wird;
Unterscheidung, unter Verwendung der erkannten Änderung in der Morphologie, zwischen einem Capture-Vorgang durch entweder den ersten oder den zweiten Stimulationskanal und einem Capture-Vorgang durch beide Stimulationskanäle;
Bestimmung einer ersten kardialen Capture-Impulsenergieniveau-Schwelle für den ersten Stimulationskanal, und einer zweiten kardialen Capture-Impulsenergieniveau-Schwelle für den zweiten Stimulationskanal; und
Ausgabe von Angaben zur ersten und zweiten kardialen Capture-Impulsenergieniveau-Schwelle an einen Benutzer oder Prozess.

12. Maschinenlesbares Medium nach Anspruch 11, einschließlich Anweisungen, die, wenn sie durch die Vorrichtung ausgeführt werden, bewirken, dass die Vorrichtung: unter Verwendung der erkannten Änderung in der Morphologie, zwischen einem Capture-Vorgang durch entweder einen oder beide Stimulationskanäle und einem Capture-Verlust durch beide Stimulationskanäle unterscheidet.

13. Maschinenlesbares Medium nach Anspruch 11 oder 12, enthaltend Anweisungen, die, wenn sie durch die Vorrichtung ausgeführt werden, bewirken, dass die Vorrichtung: eine Änderung von einem Capture-Vorgang durch einen Stimulationskanal hin zu einem Capture-Verlust durch den Stimulationskanal identifiziert, wenn ein Herzaktivitätssignal, das unter Verwendung eines Sensing-Kanals, der eine Elektrode mit dem Stimulationskanal teilt, erfasst wird, darauf hinweist, dass eine Änderung von einer Signalmorphologie, die auf ein Capture-Sensing durch eine mit einem Stimulationskanal geteilte Kathode hindeutet, hin zu einer Signalmorphologie, die darauf hindeutet, dass kein Capture-Vorgang stattfindet, erfolgt ist.

## Revendications

1. Appareil pour un couplage électrique sur une pluralité d'électrodes implantables, l'appareil comprenant :
un circuit de stimulus (405) qui est configuré pour appliquer de l'énergie impulsionnelle électrique sur au moins un premier canal de stimulation cardiaque qui inclut une première électrode de ventricule gauche (LV) (165 ; 260 ; 565) en tant que cathode et un second canal de stimulation cardiaque qui inclut une seconde électrode de LV (164 ; 262, 264 ; 560) en tant que cathode ;
un circuit de détection de signal cardiaque (410) qui est configuré pour détecter des signaux d'activité cardiaque en utilisant au moins un premier canal de détection qui inclut soit la première électrode de LV (165 ; 260 ; 565), soit la seconde électrode de LV (164 ; 262, 264 ; 560) ; et
un circuit de commande (415) qui est couplé électriquement au circuit de détection de signal cardiaque (410) et au circuit de stimulus (405), dans lequel le circuit de commande (415) inclut un sous-circuit de détection de capture (420) qui est configuré pour : initier la délivrance d'énergie impulsionnelle électrique sur à la fois le premier canal de stimulation cardiaque et le second canal de stimulation cardiaque ; détecter une dépolarisation cardiaque d'un ventricule en utilisant le premier canal de détection ; déterminer un premier seuil de niveau d'énergie impulsionnelle de capture cardiaque pour le premier canal de stimulation cardiaque et un second seuil de niveau d'énergie impulsionnelle de capture cardiaque pour le second canal de stimulation cardiaque ; et fournir des indications des premier et second seuils de niveau d'énergie impulsionnelle de capture cardiaque à un utilisateur ou à un processus ;
dans lequel le sous-circuit de détection de capture (420) est configuré pour initier une modification du niveau d'énergie impulsionnelle de l'énergie impulsionnelle électrique qui est délivrée sur à la fois le premier canal de stimulation cardiaque et le second canal de stimulation cardiaque ;
dans lequel le circuit de commande (415) inclut un sous-circuit de traitement de signal (430) qui est configuré pour identifier une modification de la morphologie d'un signal d'activité cardiaque qui est détectée en association avec une modification constituée par un passage depuis un premier niveau de l'énergie impulsionnelle électrique qui est délivrée sur à la fois le premier canal de stimulation cardiaque et le second canal de stimulation cardiaque jusqu'à un second niveau de l'énergie impulsionnelle électrique qui est délivrée sur à la fois le premier canal de stimulation cardiaque et le second canal de stimulation cardiaque ; et
dans lequel le sous-circuit de détection de capture (420) est en outre configuré pour effectuer un distinguo, en utilisant la modification identifiée de la morphologie, entre une capture cardiaque par l'un des premier et second canaux de stimulation cardiaque et une capture cardiaque par les deux canaux de stimulation cardiaque.

2. Appareil selon la revendication 1, dans lequel le sous-circuit de détection de capture (420) est configuré pour effectuer un distinguo, en utilisant la modification identifiée de la morphologie, entre une capture cardiaque par l'un ou par les deux des premier et second canaux de stimulation cardiaque et une perte de capture par les deux canaux de stimulation cardiaque.

3. Appareil selon la revendication 1, dans lequel le sous-circuit de détection de capture (420) est configuré pour identifier une modification depuis une capture par un canal de stimulation cardiaque jusqu'à une perte de capture par le canal de stimulation cardiaque lorsqu'un signal d'activité cardiaque qui est détecté en utilisant un canal de détection qui partage une électrode (265 ; 560, 565) avec le canal de stimulation cardiaque indique une modification depuis une morphologie de signal qui indique une capture qui est détectée en utilisant une cathode qui est partagée avec un canal de stimulation cardiaque jusqu'à une morphologie de signal qui indique une absence de capture cardiaque.

4. Appareil selon la revendication 1, dans lequel le sous-circuit de détection de capture (420) est configuré pour identifier une modification depuis une capture par à la fois les premier et second canaux de stimulation cardiaque jusqu'à une capture par un seul canal de stimulation cardiaque lorsqu'un signal d'activité cardiaque qui est détecté en utilisant un canal de détection qui partage une électrode (265 ; 560, 565) avec le canal de stimulation cardiaque indique une modification depuis une morphologie de signal qui indique une capture qui est détectée en utilisant une cathode qui est partagée avec un canal de stimulation cardiaque jusqu'à une morphologie de signal qui indique une capture qui est détectée en utilisant une cathode qui est indépendante d'un canal de stimulation cardiaque.

5. Appareil selon la revendication 1, dans lequel le circuit de commande (415) inclut un sous-circuit de traitement de signal (430) qui est configuré pour identifier une modification de la morphologie d'un signal d'activité cardiaque détecté qui indique une détection par cathode partagée au moyen d'un canal de détection de signal et qui survient selon une relation temporelle par rapport à la modification du niveau d'énergie impulsionnelle ; et dans lequel le sous-circuit de détection de capture (420) est configuré pour identifier une modification depuis une absence de capture par un canal de stimulation cardiaque jusqu'à une capture par le canal de stimulation cardiaque lorsque la modification de la morphologie est identifiée.

6. Appareil selon la revendication 1,
dans lequel le premier canal de détection inclut la première électrode de LV (165 ; 260 ; 565) en tant que cathode de détection ;
dans lequel le circuit de détection de signal cardiaque (410) est en outre configuré pour détecter des signaux d'activité cardiaque en utilisant un second canal de détection qui inclut la seconde électrode de LV (164 ; 262, 264 ; 560) en tant que cathode de détection ; et
dans lequel le sous-circuit de détection de capture (420) est configuré pour :
surveiller des signaux d'activité cardiaque en utilisant seulement le premier canal de détection ;
détecter une perte de capture par le premier canal de stimulation cardiaque en utilisant le premier canal de détection ;
déterminer un premier seuil de niveau d'énergie impulsionnelle de capture cardiaque pour le premier canal de stimulation cardiaque en utilisant un niveau d'énergie impulsionnelle qui est associé à la perte de capture par le premier canal de stimulation cardiaque ;
effectuer une modification au niveau de la surveillance de signaux d'activité cardiaque en utilisant seulement le second canal de détection après que la perte de capture par le premier canal de stimulation cardiaque est détectée ;
détecter une perte de capture par le second canal de stimulation cardiaque en utilisant le second canal de détection ; et
déterminer un second seuil de niveau d'énergie impulsionnelle de capture cardiaque pour le second canal de stimulation cardiaque en utilisant un niveau d'énergie impulsionnelle qui est associé à la perte de capture par le second canal de stimulation cardiaque.

7. Appareil selon la revendication 1,
dans lequel le premier canal de détection inclut la première électrode de LV (165 ; 260 ; 565) en tant que cathode de détection et est configuré pour détecter un premier signal d'activité cardiaque ;
dans lequel le circuit de détection de signal cardiaque (410) est en outre configuré pour détecter un second signal d'activité cardiaque en utilisant un second canal de détection qui inclut la seconde électrode de LV (164 ; 262, 264 ; 560) en tant que cathode de détection ; et
dans lequel le sous-circuit de détection de capture (420) est configuré pour :
détecter une modification entre une capture et une perte de capture par le premier canal de stimulation cardiaque en utilisant le premier canal d'activité cardiaque et déterminer le premier seuil de niveau d'énergie impulsionnelle de capture cardiaque pour le premier canal de stimulation cardiaque en utilisant un niveau d'énergie impulsionnelle qui est associé à la modification détectée ; et
détecter une modification entre une capture et une perte de capture par le second canal de stimulation cardiaque en utilisant le second canal d'activité cardiaque et déterminer le second seuil de niveau d'énergie impulsionnelle de capture cardiaque pour le second canal de stimulation cardiaque en utilisant un niveau d'énergie impulsionnelle qui est associé à la modification détectée.

8. Appareil selon la revendication 1, incluant un boîtier implantable (150 ; 282 ; 582), dans lequel le premier canal de détection inclut la première électrode de LV (165 ; 260 ; 565) en tant que cathode de détection et une électrode qui est formée sur le boîtier implantable (150 ; 282 ; 582) en tant qu'anode de détection.

9. Appareil selon la revendication 1, dans lequel le circuit de détection de signal cardiaque (410) est configuré pour être couplé électriquement à une électrode de ventricule droit (RV), et dans lequel le premier canal de détection inclut la première électrode de LV (165 ; 260 ; 565) en tant que cathode de détection et l'électrode de RV (175 ; 275 ; 575) en tant qu'anode de détection.

10. Appareil selon l'une quelconque ou une combinaison des revendications 1 à 9, dans lequel le circuit de stimulus (405) est configuré pour appliquer de l'énergie impulsionnelle électrique sur plus de deux canaux de stimulation cardiaque, dans lequel chaque canal de stimulation cardiaque inclut une électrode de LV différente (164, 165 ; 260, 262, 264 ; 560, 565), et dans lequel le sous-circuit de détection de capture (420) est configuré pour :
sélectionner une paire des canaux de stimulation cardiaque ;
sélectionner un canal de détection de signal cardiaque qui inclut une électrode de LV (164, 165 ; 260, 262, 264 ; 560, 565) à partir de la paire sélectionnée de canaux de stimulation cardiaque ;
déterminer des seuils de niveau d'énergie impulsionnelle de capture cardiaque pour les canaux de stimulation cardiaque sélectionnés ; et
continuer à sélectionner des paires des canaux de stimulation cardiaque et à déterminer des seuils de niveau d'énergie impulsionnelle de capture cardiaque pour les paires sélectionnées jusqu'à ce qu'un seuil de niveau d'énergie impulsionnelle de capture cardiaque soit déterminé pour chacun des canaux de stimulation cardiaque.

11. Support pouvant être lu par une machine et incluant des instructions qui, lorsqu'elles sont réalisées par l'appareil selon l'une quelconque des revendications 1 à 10, forcent l'appareil à effectuer les actions comprenant :
la délivrance d'énergie impulsionnelle électrique sur à la fois au moins un premier canal de stimulation cardiaque de l'appareil qui inclut une première électrode de ventricule gauche (LV) (165 ; 260 ; 565) en tant que cathode et un second canal de stimulation cardiaque de l'appareil qui inclut une seconde électrode de LV (164 ; 262, 264 ; 560) en tant que cathode ;
la détection d'une dépolarisation cardiaque d'un ventricule en utilisant au moins un premier canal de détection qui inclut soit la première électrode de LV (165 ; 260 ; 565), soit la seconde électrode de LV (164 ; 262, 264 ; 560) ;
la modification d'un niveau d'énergie impulsionnelle de l'énergie impulsionnelle électrique qui est délivrée sur à la fois les premier et second canaux de stimulation cardiaque ;
l'identification d'une modification de la morphologie d'un signal d'activité cardiaque qui est détectée en association avec une modification constituée par un passage depuis un premier niveau de l'énergie impulsionnelle électrique qui est délivrée sur à la fois le premier canal de stimulation cardiaque et le second canal de stimulation cardiaque jusqu'à un second niveau de l'énergie impulsionnelle électrique qui est délivrée sur à la fois le premier canal de stimulation cardiaque et le second canal de stimulation cardiaque ;
la réalisation d'un distinguo, en utilisant la modification identifiée de la morphologie, entre une capture cardiaque par l'un des premier et second canaux de stimulation cardiaque et une capture cardiaque par les deux canaux de simulation cardiaque ;
la détermination d'un premier seuil de niveau d'énergie impulsionnelle de capture cardiaque pour le premier canal de stimulation cardiaque et d'un second seuil de niveau d'énergie impulsionnelle de capture cardiaque pour le second canal de stimulation cardiaque ; et
la fourniture d'indications des premier et second seuils de niveau d'énergie impulsionnelle de capture cardiaque à un utilisateur ou à un processus.

12. Support pouvant être lu par une machine selon la revendication 11 et incluant des instructions qui, lorsqu'elles sont réalisées par l'appareil, forcent l'appareil à : effectuer un distinguo, en utilisant la modification identifiée de la morphologie, entre une capture cardiaque par l'un ou par les deux des premier et second canaux de stimulation cardiaque et une perte de capture par les deux canaux de stimulation cardiaque.

13. Support pouvant être lu par une machine selon la revendication 11 ou 12, incluant des instructions qui, lorsqu'elles sont réalisées par l'appareil, forcent l'appareil à : identifier une modification depuis une capture par un canal de stimulation cardiaque jusqu'à une perte de capture par le canal de stimulation cardiaque lorsqu'un signal d'activité cardiaque qui est détecté en utilisant un canal de détection qui partage une électrode avec le canal de stimulation cardiaque indique une modification depuis une morphologie de signal qui indique une capture qui est détectée en utilisant une cathode qui est partagée avec un canal de stimulation cardiaque jusqu'à une morphologie de signal qui indique une absence de capture cardiaque.
